# EUROPEAN PATENT SPECIFICATION

(11) **EP 3 110 457 B1**
(45) Date of publication and mention of the grant of the patent: **21.03.2018**
(21) Application number: 15700506.7
(22) Date of filing: 21.01.2015
(51) Int. Cl.: A61L 2/08, A61L 2/28, B65B 55/08, H01J 33/04

(54) **DEVICE AND METHOD FOR ELECTRON BEAM STERILIZATION COMPRISING TEMPERATURE MEASUREMENT DEVICE, TEMPERATURE CORRELATED TO RADIATION INTENSITY**
VORRICHTUNG UND VERFAHREN ZUR ELEKTRONENSTRAHLSTERILISATION MIT TEMPERATURMESSUNGSVORRICHTUNG IN KORRELATION ZUR STRAHLUNGSINTENSITÄT
DISPOSITIF ET PROCÉDÉ POUR LA STÉRILISATION PAR FAISCEAU D'ÉLECTRONS COMPRENANT UN DISPOSITIF DE MESURE DE TEMPÉRATURE, LA TEMPÉRATURE CORRESPONDANT À UNE INTENSITÉ DE RAYONNEMENT

(30) Priority: 26.02.2014 SE 1450224
(43) Date of publication of application: 04.01.2017
(73) Proprietor: Tetra Laval Holdings & Finance SA, 1009 Pully (CH)
(72) Inventor: OMRANE, Alaa, S-226 55 Lund (SE)
(74) Representative: Tetra Pak - Patent Attorneys SE
(86) International application number: PCT/EP2015/051076
(87) International publication number: WO 2015/128117

(56) References cited:
- WO-A1-02/072160
- WO-A1-2007/050010
- WO-A1-2011/011079
- US-A1- 2007 290 148

## Description

The invention relates to a device and method for sterilization of a packaging container or a packaging material by electron beam irradiation, as set out in the appended set of claims. Within for example the food industry, it is common practice to pack liquid and partly liquid food products in packaging containers manufactured from polymer materials such as for example PET, or from a packaging laminate comprising a core layer of paper or paperboard and one or more barrier layers of, for example, polymer material or aluminium foil. One packaging type is the "carton bottle" manufactured in a filling machine in that packaging blanks of the above-described packaging laminate are formed and sealed as a sleeve. Said sleeve is closed in one end in that a top of thermoplastic material is injection moulded directly on the sleeve end portion. The sheets of packaging laminate may be cut from a magazine reel of packaging laminate.

When the top is finished the packaging container is ready to be filled with product through the still open bottom, and then sealed and finally folded. Before the filling operation the packaging container undergoes treatment. If distribution and storage is to be made in chilled temperature the packaging container is disinfected, whereas if distribution and storage is to be made in ambient temperature, the packaging container needs to be sterilized. Sterilization is a term referring to any process that eliminates or kills microbial life, including transmissible agents such as for example fungi, bacteria, viruses and spores, which may be present on a surface of the packaging material or in a product. In the (food) packaging industry this is generally referred to as aseptic packaging, i. e. packaging sterilized products in sterilized packaging containers, i. e. keeping both the product and the packaging container free form living germs and microorganisms, so that the freshness of the product can be preserved without special cooling requirements, i. e. so that sterility can be maintained inside a packaging container although it is stored in ambient temperature. In this context the term "commercially sterile" is also commonly used and means in general the absence of microorganisms capable of growing in the food at normal non-refrigerated conditions at which the food is likely to be held during manufacture, distribution and storage. In this patent application the word "sterile" refers to a condition being at least commercially sterile.

One way to sterilize such packaging containers is to irradiate it by means of a low voltage electron beam emitted from an electron beam emitter. An example of linear irradiation by electron beam of ready-to-fill packaging containers is disclosed in the international patent publication WO 2005/002973. Other examples of irradiation of packaging containers, in these cases PET bottles, are described in for example WO 2011/011079 and EP 2 371 397, the latter describing a rotary system. In these systems emitters are used having a diameter small enough to be passed through a neck portion of the bottles. Low voltage electron beam can also be used for sterilizing a web of packaging material. The web is transported in between two opposed electron beam emitters having their electron exit windows facing the surfaces of the web. Web sterilization is for example described in the international publication WO2004/110868.

Electron beam sterilization devices, or emitters, are known which can be inserted into a packaging container for sterilizing the interior of the packaging container. Moreover, there are electron beam sterilization devices for sterilizing exterior surfaces of a packaging container.

In order to monitor correct operation of the electron beam emitters, and thereby being able to secure sterility, it is common practise to perform dosimetry tests. These tests are made regularly, generally daily, throughout the lifetime of the electron beam emitter. In general, dosimetry tests involve adding a dosimeter means, i.e. a patch reacting on radiation exposure, to a packaging container to measure if a correct absorbed dose is obtained during radiation. At the same time measurements of voltage and current are made in the electron beam emitter. The current over the filament is measured by comparing the current fed to the filament and current leaving the filament. In this way it is possible to determine the amount of electrons emitted from the filament. In addition, the voltage, i.e. the electric potential, between the electron exit window and the filament is measured. The measured value of voltage and current is then used as a set value during production of packaging containers. The current and voltage are continuously monitored during production, and as long as the value is not lower than the set value it is assumed that the packaging containers receive the correct dose. WO 02/072160 is a sterilization system for sterilizing medical product that comprises a radiation source, calorimeter and calorimeter controller, according to the preamble of claim 1. It is an object of the present invention to provide an electron beam sterilization device and a method for sterilizing a packaging container or a web of packaging material allowing for a particularly reliable sterilization in that on-line measurement and control of the functionality of the electron beam sterilization device is improved.

The object is solved according to the invention with an electron beam sterilizing device according to claim 1 and a method for electron beam sterilization according to claim 9. Preferred embodiments are defined in the dependent claims and the following description, in particular taken together with the attached drawings.

The electron beam sterilizing device according to the invention is as defined in claim 1. The method according to the invention is a method for sterilizing a packaging container or a packaging material web by electron beam irradiation, as defined in dependent claim 9. In order to obtain a suitable sterilization, the irradiation dose of the electron beam sterilizing device has to be kept within a given range. If the delivered dose is too small the sterilization might not be adequate. If the dose is too high, the packaging material might be affected negatively. It is therefore generally desirable to determine the irradiation dose, in particular the intensity of the electron beam of an electron beam sterilizing device, when operating the electron beam sterilizing device.

A basic idea of the invention is to provide a device and a method for determining the irradiation dose or intensity of the electron beam (output dose/electron beam energy output) or the electron energy delivered by the electron beam of an electron beam sterilizing device. The irradiation dose or electron beam dose is determined through a measurement of a temperature in a travelling path of the electron beam. When the electron beam, i.e. the electrons emitted by the electron beam generator, hits an object, heat is generated and the respective object is heated. By correlating the temperature of the object to the output dose of the electron beam sterilizing device, the temperature measurement device can be used as a dose sensor of the electron beam sterilizing device. The temperature measurement takes place during the operation of the electron beam sterilizing device, i.e. while an electron beam is generated and the electron beam passes through the electron exit window. The electron beam path is particularly a path extending from the electron beam generator towards the packaging container or packaging material web to be sterilized, i.e. from the electron beam generator towards and through the electron exit window.

The temperature can generally be measured at any location in the travelling path (electron beam path), for example between the electron beam generator and the electron exit window. In one or more embodiments, however, the temperature is measured at the electron exit window and the measurement device is adapted to measure a temperature of or at the electron exit window. The temperature of the electron exit window depends on the electron energy or irradiation dose or beam intensity and, therefore, the irradiation dose can be determined by determining the temperature of the electron exit window. When the electron beam passes through the electron exit window, the energy of the electron beam is partly transferred to the electron exit window. The electron exit window therefore heats up when the electron beam sterilizing device is operated, and the temperature of the electron exit window is directly related to the irradiation dose, in particular the electron current, or electron energy, emitted by the electron beam sterilizing device (emitter). Measurement of the temperature of the electron exit window therefore provides a simple way of determining the irradiation dose. The temperature measurement device can, therefore, be used to monitor the irradiation dose. The temperature measurement device comprises two temperature sensors arranged at the electron exit window. The temperature sensor, which can for example be a thermocouple, preferably contacts the electron exit window and detects the temperature of at least one specified position of the electron exit window, in particular through direct contact. The temperature measurement device comprises at least two temperature sensors. The temperature sensors may be located at different positions of the electron exit window in order to detect uniformity of the electron beam over the electron exit window. The temperature measurement device is situated in the internal space of the housing, in the travelling path of the electrons, i.e. between the electron generator and the electron exit window. The temperature measurement device is at least partially covered by a medium adapted to create heat when impinged by electrons. The medium may be a ceramic material. A similar temperature device may be used outside the electron exit window, in positions where the temperature device is not in direct contact with the electron exit window, still being in the travelling path of the electrons.

In one embodiment of the invention the electron exit window comprises a support structure and a foil which is transmissible for electrons, and the temperature measurement device, in particular the temperature sensor, is adapted to measure a temperature of the support structure and/or the foil. This embodiment is based on the fact that the energy delivered by the electron beam sterilizing device, or emitter, is partially and linearly deposited on both the vacuum window (foil) and its support. Therefore, measuring the temperature on these items provides a simple way of determining the electron current itself. The support element may in particular be a grid-like structure, for example made of copper, having a plurality of free spaces or holes through which the electrons can pass. The foil is permeable for electrons and may be a metallic foil, such as for example a titanium foil. The thickness of the foil may be in the range of 4-12 micrometers. The foil covers the support structure. For a measurement of the temperature of the support structure and/or the foil, a sensing head of the measurement device, or temperature sensor, can in one embodiment directly contact the support structure and/or the foil.

In another embodiment of the invention the temperature measurement device, in particular the temperature sensor, is arranged on and/or within the support structure. The positioning of the temperature sensor on and/or within the support structure allows for a non-intrusive in situ dose/temperature measurement, in particular if the temperature sensor does not enter the free spaces of the support structure. The temperature sensor is preferably arranged such that it does not interfere or block the electron beam passing through the free spaces of the support structure. The temperature measurement device, in particular the temperature sensor, is preferably arranged on a surface of the supporting structure facing the internal space or on a surface opposite the internal space, so as to avoid blocking of the free spaces in the support structure. In another embodiment, the temperature sensor can also be embedded in the material of the support structure.

In order to obtain a particularly reliable and/or predictable relationship between the measured temperature and the irradiation dose, the temperature measurement device, in particular the temperature sensor, is arranged so as to measure a temperature in a central portion of the electron exit window. In particular, if the electron exit window is substantially symmetrical with regard to the central portion or point, the temperature at this point is a reliable measure of the electron current. In one embodiment of the invention, the electron exit window is circular. The temperature measurement device is adapted to measure temperatures at at least two different positions of the electron exit window. Through temperature measurements at more than one position of the electron exit window, the reliability of the determination of the emitted electron irradiation dose can be enhanced. The temperature measurement device can comprise two or more separate temperature sensors for measuring temperatures at distinct positions at the electron exit window. If a field of temperature sensors is provided, it is also possible to determine a spatial radiation profile of the electron beam sterilization device.

In one embodiment of the invention, the temperature measurement device comprises a thermocouple. A thermocouple provides a sufficient accuracy as needed for the present application and a reliable and cost efficient way of measuring temperatures.

In another embodiment of the invention the temperature measurement device comprises a non-contacting temperature sensor for measuring a temperature of the electron exit window. For example, a thermometer for measuring thermal radiation can be used. The thermal radiation is used for determining the temperature of the electron exit window. A known device of this type is a pyrometer. The pyrometer can for example be placed outside the housing of the electron beam sterilizing device and such that it is passed by the electron beam sterilizing device between successive sterilizations of packaging containers. The electron beam sterilizing device is preferably kept operating between the successive sterilizations so that a stable temperature at the electron exit window is provided, i.e. the temperature of the electron exit window is substantially constant over time.

The reliability of the determination of the electron irradiation dose based on a temperature measurement of only a portion of the electron exit window also depends on the temperature profile at the electron exit window generated by the electrons passing through the electron exit window. The more predictable and the more evenly distributed the temperature profile, the more accurate the determination of the electron irradiation dose. It is therefore preferred according to the invention to use an electron beam generator having a predictable and/or homogeneous electron emitting profile.

In one embodiment of the invention, the electron beam sterilizing device comprises an electron irradiation dose calculator for calculating an electron irradiation dose, in particular an electron current, based on the measured temperature. The electron irradiation dose calculator uses calibration data defining a relationship between the measured temperature, in particular at a specific position of the electron exit window, and the electron irradiation dose emanating from the electron exit window.

In yet another embodiment of the invention the electron beam sterilizing device comprises a display for displaying the measured temperature and/or for displaying the electron irradiation dose determined based on the measured temperature. The display is operationally connected to the temperature sensor and/or the electron irradiation dose calculator.

Another embodiment is defined by an alarm device which is adapted to trigger an alarm signal when the measured temperature exceeds or falls below a predetermined threshold. The alarm device is operationally connected to the temperature measurement device and may be adapted to emit an acoustic and/or visual alarm signal. A maximum temperature at the electron exit window can be in the range of 300°C to 400°C. Alternatively, a feedback signal is generated and sent to an irradiation control module if the temperature decreases below a chosen threshold temperature. An irradiation control module is a module controlling various processes with regard to the sterilization. A certain feedback signal may be arranged to generate appropriate measures to be taken.

In one or more embodiments of the method according to the invention an electron irradiation dose is determined based on the measured temperature. The determination of the electron irradiation dose preferably uses calibration data indicative of a relationship between the measured temperature, in particular at a specific position of the electron exit window, and the overall electron irradiation dose of the electron exit window. The measurement of the temperature takes place during operation of the electron beam sterilization device, i.e. while the electron beam passes through the electron exit window.

In the following, the invention will be further described in connection with the attached drawings, in which:
- Fig. 1:: shows an embodiment of an electron beam sterilization device according to the invention;
- Fig. 2:: shows a sterilization process of sterilizing an interior volume of a packaging container;
- Fig. 3:: shows another embodiment of an electron exit window according to the invention;
- Fig. 4:: shows a calibration curve for determining an electron current based on a measured temperature at an electron exit window.
- Fig. 5:: shows a circular electron exit window and a rectangular electron exit window and the position of several temperature sensors.

Equal or corresponding elements are denominated with the same reference numerals in all figures. Features described in connection with different figures can be combined as long as technically possible.

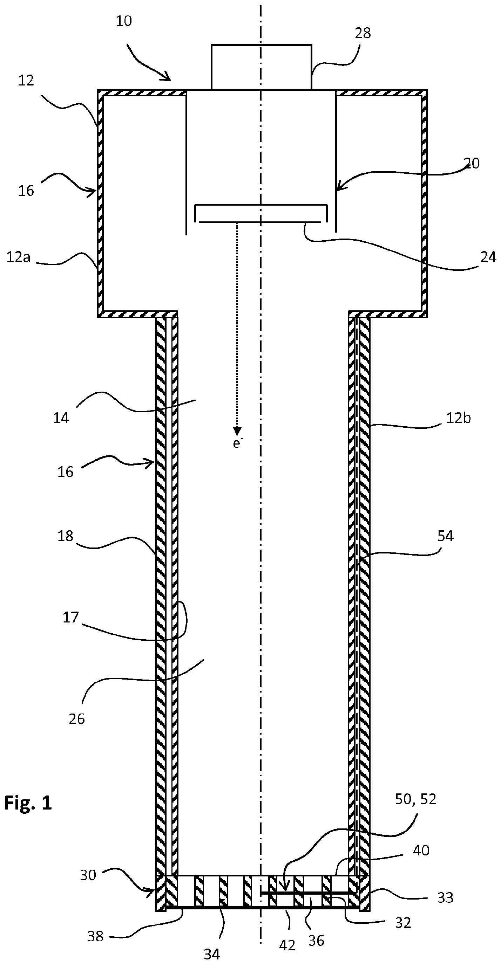

Fig. 1 shows an electron beam sterilizing device 10 according to the invention for an interior sterilization of a packaging container 60, in particular a drug or food container.

The electron beam sterilizing device 10 comprises a housing 12 enclosing an internal space 14, in particular a vacuum space or vacuum chamber. An electron beam generator 20 is arranged in the housing 12, i.e. in the internal space 14. An electron exit window 30, which is permeable or transmissible for electrons, is arranged at an end surface of the housing 12. The housing 12 has a first part 12a provided with the electron generator 20, and a second part 12b provided with the electron exit window 30. The interior surface of a packaging container is sterilized by providing a relative movement between the electron beam sterilizing device 10 and the packaging container. Thus, the first part 12a has a larger diameter than the second part 12b, and the second part 12b has a diameter small enough to be inserted through an opening of the packaging container.

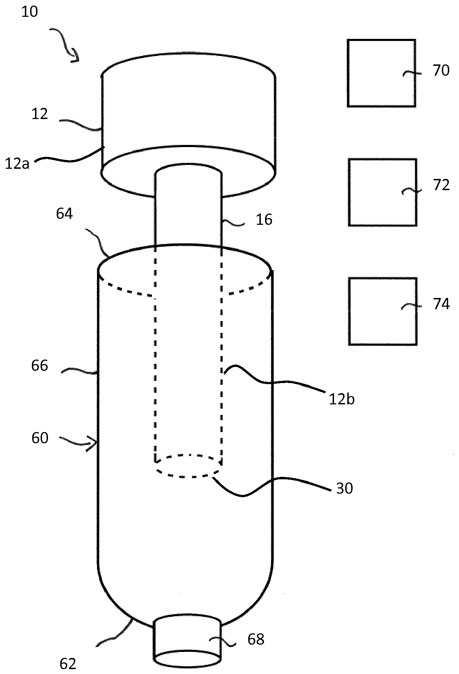

The electron beam generator 20 comprises a filament 24 which, when heated to an elevated temperature, in particular in the order of 2000°C, is caused to emit a cloud of electrons. The filament 24 can for example be made of tungsten. The electrons are accelerated in an electron acceleration zone 26 towards the electron exit window 30 by means of a voltage potential between the filament 24 (cathode) and the electron exit window 30 (anode). Due to the energy of the electrons, the electrons pass through the electron exit window 30 towards a target area. The target area is in this case the inside of a packaging container 60 to be sterilized, see Figure 2. The acceleration of the electrons in the electron acceleration zone 26, Figure 1, is achieved by a high-voltage field between the electron beam generator 20 and the electron exit window 30. The potential difference in the high-voltage field may be in the order of 75 kV to 150 kV. The filament 24 is coupled to a high voltage supply through a connector 28.

The housing 12 has a substantially longitudinal shape so as to be at least partially inserted into a packaging container 60 to be sterilized, as shown in Figure 2. The electron beam sterilization device 10 can for example be inserted into the packaging container 60 through an open top or bottom end of the packaging container 60. In the embodiment shown in Figure 2, the packaging container 60 has an upper side 62, a lower side 64 and a side wall 66 of a substantially tubular shape between the upper side 62 and the lower side 64. The electron beam sterilization device 10 is inserted into the packaging container 60 through the open lower side 64 of the packaging container 60. Alternatively, the electron beam sterilization device 10 may, however, also be inserted into the packaging container 60 through an opening in the upper side 62 of the packaging container 60. The upper side 62 can be spout, as shown in Figure 2, which after filling can be closed by for instance a screw cap.

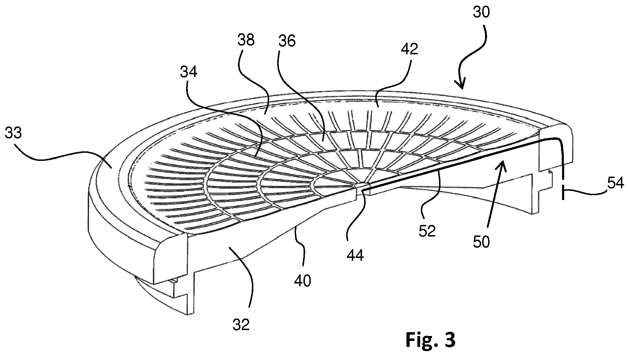

The electron exit window 30, best seen in Figure 3, has a circular shape and comprises a grid-like support structure 32, for example made of aluminium or copper, and a metallic foil 38 which is transmissible for electrons. The metallic foil 38 is supported by the support structure 32 and can have a thickness in the order of 4 to 12 micrometers. The support structure 32, for example a grid or perforated plate, comprises a plurality of ribs 34 and a plurality of apertures 36 between the ribs 34. The ribs 34 extend at least partly in a radial direction of the electron exit window 30. The electron exit window 30 has an inner side 40 facing towards the internal space 14 or interior of the housing 12 and an outer side 42 facing, in use, towards the packaging container 60 to be sterilized.

For measuring a temperature at at least one position of the electron exit window 30 the electron beam sterilization device 10 comprises a temperature measurement device 50. The temperature measurement device 50 comprises a temperature sensor 52, for example a thermocouple, positioned at the support structure 32 of the electron exit window 30. In the Figure 3 embodiment the temperature sensor 52 is arranged at or within one of the ribs 34 of the support structure 32, preferably a rib 34 extending in a radial direction. In order not to obstruct the apertures 36 of the support structure 32, the temperature sensor 52 can either be integrated into one of the ribs 34 or arranged on a surface of a rib 34, preferably on the outer side 42 or the inner side 40 of the electron exit window 30 or the support structure 32. Alternatively, and as is shown in Figure 1, the temperature sensor 52 may extend partly through apertures 36 and ribs 34.

The temperature measurement device 50 further comprises a cable 54 for connecting the temperature sensor 52 to a signal converter, in particular for converting a voltage value to a temperature value. The cable 54 is routed along a side wall 16 of the housing 14, for example as shown in Fig. 1 where the cable 54 is illustrated as a dashed line. As can be seen in Fig. 1, the side wall 16 is composed of an inner wall 17 and an outer wall 18, and the cable 54 is routed through an interior space between the inner wall 17 and the outer wall 18. However, other arrangements are also possible.

Figure 3 shows an embodiment in which the temperature sensor 52 of the temperature measurement device 50 extends at or within the support structure 32. It extends in a radial direction between a central position 44 of the electron exit window 30 and an outer support ring 33 of the support structure 32. The temperature sensor 52 is a thermocouple comprising two different metals as is generally known in the art. A diameter of the thermocouple can be in the range of 15 to 35 micrometres, in particular 20 to 30 micrometres. The measuring point of the thermocouple is arranged in a central area (central position 44) of the electron exit window 30 and contacts the support structure 32 and/or the foil 38. For generating a homogeneous temperature profile at the electron exit window 30, the ribs 34 of the support structure 32 may have a varying thickness between the central position 44 of the support structure 32 and a radially outer portion of the support structure 32.

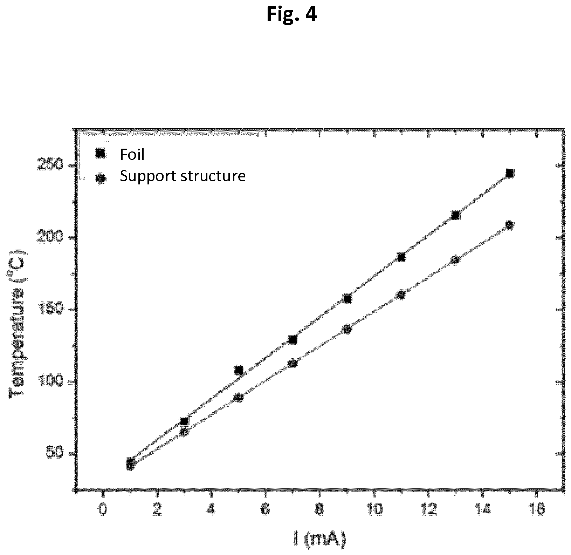

Figure 4 shows a calibration curve for calibrating the temperature measurement device 50. On the x-coordinate the electron current I is shown (mA) and on the y-coordinate the temperature is shown (°C). There is a linear relationship between the temperature and the electron current (irradiation dose). It can be seen that the temperature of the foil is generally higher than the temperature of the support structure. By means of this calibration curve, the electron current and/or the irradiation dose can be determined based on a measured temperature.

An electron irradiation dose calculator 70 is provided for calculating an irradiation dose or beam intensity based on the measured temperature. The measured temperature and/or the irradiation dose can be displayed on a display 72. An alarm device 74 is arranged for providing an alarm signal if the measured temperature exceeds or falls below a predetermined threshold temperature.

The invention provides an effective way of determining the electron current of an electron beam emitter used for sterilizing packaging containers, in particular for use in the drug or food industry. The device is robust and can be manufactured at low costs. A thin thermocouple can be used and made sensitive to temperature variations as required. The measured temperature is directly related to the electron current so that the temperature is a direct measure of the irradiation dose. A simple calibration can be used for determining the irradiation dose. The temperature measurement device can therefore be used as a dose sensor and/or an alarm monitor.

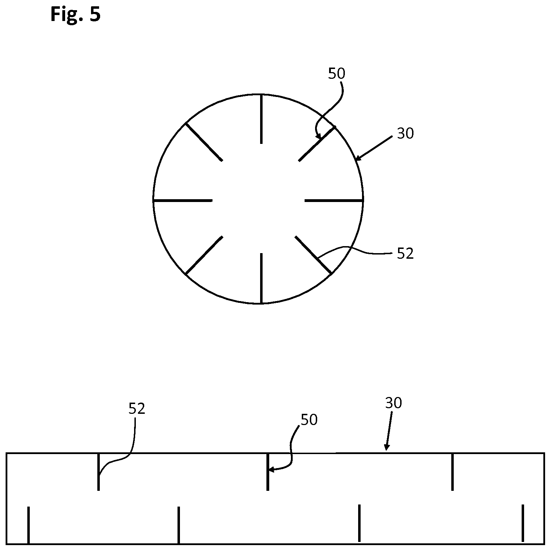

An exemplary temperature measurement device 50 may comprise several temperature sensors 52 distributed over the electron exit window in order to be able to detect also the uniformity, and any irregularities of the uniformity, of the electron beam. The upper view of Figure 5 shows in a very schematic way a circular electron exit window 30. A temperature measurement device 50 comprises eight temperature sensors 52 arranged in or at the electron exit window, and are able to measure the temperature at eight different positions of the window. The temperature sensors are arranged equally distributed along the perimeter of the electron exit window and pointing in a direction towards the centre of the electron exit window 30. They do not reach all the way into the centre, but measure a distance from the centre. This is one exemplary arrangement suitable for electron beams being ring-shaped, i.e. when intensity in the centre of the electron beam is less than near the perimeter of the electron beam. The lower view of Figure 5 shows a rectangular electron exit window 30 and the position of several temperature sensors 52 substantially equally distributed over the length of the window.

It has been described that the temperature measurement device may be provided in an electron beam sterilizing device for sterilization of the interior surfaces of packaging containers. It should be understood that the temperature measurement device of the invention is also suitable for electron beam sterilizing devices used for sterilization of packaging material webs or exterior surfaces of packaging containers. Such sterilization is described in for example the international publication WO2004/110868. One example of an electron beam emitter suited for web sterilization and exterior surface sterilization of packaging containers is shown in the international publication WO2013/004565. One or more temperature measurement devices are fitted in the support structure. The support structure may be of the kind described in the international publication WO2011/096874.

### Reference numerals:

- 10: electron beam sterilizing device
- 12: housing
- 14: internal space
- 16: side wall
- 17: inner wall
- 18: outer wall
- 20: electron beam generator
- 24: filament
- 26: electron acceleration zone
- 28: connector
- 30: electron exit window
- 32: support structure
- 33: support ring
- 34: rib
- 36: aperture
- 38: foil
- 40: inner side
- 42: outer side
- 44: central position
- 50: temperature measurement device
- 52: temperature sensor
- 54: cable
- 60: packaging container
- 62: upper side
- 64: lower side
- 66: side wall
- 68: filling hole
- 70: electron irradiation dose calculator
- 72: display
- 74: alarm device

## Claims

1. Electron beam sterilizing device for sterilizing a packaging container (60) or a packaging material web by electron beam irradiation, the electron beam sterilizing device (10) comprising:
a housing (12) enclosing an internal space (14) and
an electron beam generator (20) arranged in the internal space (14) for generating an electron beam,
wherein the housing (12) comprises an electron exit window (30),
wherein a temperature measurement device (50) is provided for measuring a temperature in a travelling path of the electron beam, said temperature being a measure of the electron energy delivered of the electron beam,
**characterized in that** the temperature measurement device (50) comprises at least two temperature sensors located at different positions of the electron exit window, and that each temperature sensor is adapted to measure a temperature.

2. Electron beam sterilizing device according to claim 1,
**characterized in that**
the temperature measurement device (50) is adapted to measure a temperature of the electron exit window (30).

3. Electron beam sterilizing device according to claim 1 or 2,
**characterized in that**
the temperature measurement device (50) comprises two ore more separate temperature sensors for measuring temperatures at distinct positions at the electron exit window (30).

4. Electron beam sterilizing device according to one of the claims 1 - 3,
**characterized in that**
the electron exit window (30) comprises a support structure (32) and a foil (38) which is transmissible for electrons, and
the temperature measurement device (50) is adapted to measure a temperature of the support structure (32) and/or the foil (38).

5. Electron beam sterilizing device according to one of the claims 1 - 4,
**characterized in that**
the electron exit window (30) comprises a support structure (32) for supporting a foil (38) which is transmissible for electrons, and
the temperature measurement device (50) is arranged on and/or within the support structure (32).

6. Electron beam sterilizing device according to one of the claims 1 - 5,
**characterized in that**
the temperature measurement device (50) is arranged so as to measure a temperature in a central portion of the electron exit window (30).

7. Electron beam sterilizing device according to one of the claims 1 - 6,
**characterized in that**
the temperature measurement device (50) comprises at least one thermocouple.

8. Electron beam sterilizing device according to one of the claims 1 - 7,
**characterized in that**
the temperature measurement device (50) comprises at least one non-contacting temperature sensor (52) for measuring a temperature of the electron exit window (30).

9. Method for sterilizing a packaging container (60) or a packaging material web by electron beam irradiation with an electron beam sterilizing device (10) according to one of the claims 1 - 8,
wherein the method comprises the steps of:
generating an electron beam in a housing,
making the electron beam exiting (30) the housing through an electron exit window, and
measuring a temperature in a travelling path of the electron beam as a measure of the electron energy delivered by the electron beam measuring a temperature at each of at least two temperature sensors located at different positions of the electron exit window.

## Patentansprüche

1. Elektronenstrahl-Sterilisationsvorrichtung zum Sterilisieren eines Verpackungsbehälters (60) oder einer Verpackungsmaterialbahn durch Bestrahlung mit Elektronenstrahlen, wobei die Elektronenstrahl-Sterilisationsvorrichtung (10) umfasst:
ein Gehäuse (12), das einen Innenraum (14) umgibt;
und
einen Elektronenstrahlgenerator (20), der im Innenraum (14) angeordnet ist, zum Erzeugen eines Elektronenstrahls,
wobei das Gehäuse (12) ein Elektronenaustrittsfenster (30) umfasst,
wobei eine Temperaturmessvorrichtung (50) zum Messen einer Temperatur in einem Laufweg des Elektronenstrahls vorgesehen ist, die Temperatur ein Maß der Elektronenenergie ist, die vom Elektronenstrahl abgegeben wird, **dadurch gekennzeichnet, dass** die Temperaturmessvorrichtung (50) mindestens zwei Temperatursensoren umfasst, die sich in verschiedenen Positionen des Elektronenaustrittsfensters befinden, und dass jeder Temperatursensor zum Messen einer Temperatur ausgelegt ist.

2. Elektronenstrahl-Sterilisationsvorrichtung nach Anspruch 1,
**dadurch gekennzeichnet, dass**
die Temperaturmessvorrichtung (50) zum Messen einer Temperatur des Elektronenaustrittsfensters (30) ausgelegt ist.

3. Elektronenstrahl-Sterilisationsvorrichtung nach Anspruch 1 oder 2,
**dadurch gekennzeichnet, dass**
die Temperaturmessvorrichtung (50) zwei oder mehr separate Temperatursensoren zum Messen von Temperaturen in verschiedenen Positionen am Elektronenaustrittsfenster (30) umfasst.

4. Elektronenstrahl-Sterilisationsvorrichtung nach einem der Ansprüche 1 bis 3,
**dadurch gekennzeichnet, dass**
das Elektronenstrahlfenster (30) eine Tragstruktur (32) und eine Folie (38) umfasst, die für Elektronen durchlässig ist, und
die Temperaturmessvorrichtung (50) zum Messen einer Temperatur der Tragstruktur (32) und/oder der Folie (38) ausgelegt ist.

5. Elektronenstrahl-Sterilisationsvorrichtung nach einem der Ansprüche 1 bis 4,
**dadurch gekennzeichnet, dass**
das Elektronenaustrittsfenster (30) eine Tragstruktur (32) zum Tragen einer Folie (38) umfasst, die für Elektronen durchlässig ist, und
die Temperaturmessvorrichtung (50) auf und/oder innerhalb der Tragstruktur (32) angeordnet ist.

6. Elektronenstrahl-Sterilisationsvorrichtung nach einem der Ansprüche 1 bis 5,
**dadurch gekennzeichnet, dass**
die Temperaturmessvorrichtung (50) zum Messen einer Temperatur in einem mittleren Abschnitt des Elektronenaustrittsfensters (30) ausgelegt ist.

7. Elektronenstrahl-Sterilisationsvorrichtung nach einem der Ansprüche 1 bis 6,
**dadurch gekennzeichnet, dass**
die Temperaturmessvorrichtung (50) mindestens ein Thermoelement umfasst.

8. Elektronenstrahl-Sterilisationsvorrichtung nach einem der Ansprüche 1 bis 7,
**dadurch gekennzeichnet, dass**
die Temperaturmessvorrichtung (50) mindestens einen kontaktlosen Temperatursensor (52) zum Messen einer Temperatur des Elektronenaustrittsfensters (30) umfasst.

9. Verfahren zur Sterilisation eines Verpackungsbehälters (60) oder einer Verpackungsmaterialbahn durch Bestrahlung mit Elektronenstrahlen mit einer Elektronenstrahl-Sterilisationsvorrichtung (10) nach einem der Ansprüche 1 bis 8,
wobei das Verfahren die folgenden Schritte umfasst:
Erzeugen eines Elektronenstrahls in einem Gehäuse,
Bewirken, dass der Elektronenstrahl durch ein Elektronenaustrittsfenster aus dem Gehäuse austritt, und
Messen einer Temperatur in einem Laufweg des Elektronenstrahls als ein Maß der Elektronenenergie, die durch den Elektronenstrahl abgegeben wird,
Messen einer Temperatur an jedem von mindestens zwei Temperatursensoren, die sich in verschiedenen Positionen des Elektronenaustrittsfensters befinden.

## Revendications

1. Dispositif de stérilisation par faisceau d'électrons destiné à stériliser un récipient d'emballage (60) ou une bande de matériau d'emballage par irradiation avec un faisceau d'électrons, le dispositif de stérilisation par faisceau d'électrons (10) comprenant :
un boîtier (12) enfermant un espace interne (14)
et
un générateur de faisceau d'électrons (20) disposé dans l'espace interne (14) destiné à générer un faisceau d'électrons,
dans lequel le boîtier (12) comprend une fenêtre de sortie d'électrons (30),
dans lequel un dispositif de mesure de température (50) est prévu pour mesurer une température sur une trajectoire de déplacement du faisceau d'électrons, ladite température étant une mesure de l'énergie des électrons délivrée du faisceau d'électrons,
**caractérisé en ce que** le dispositif de mesure de température (50) comprend au moins deux capteurs de température situés à des positions différentes de la fenêtre de sortie d'électrons, et **en ce que** chaque capteur de température est adapté pour mesurer une température.

2. Dispositif de stérilisation par faisceau d'électrons selon la revendication 1,
**caractérisé en ce que**
le dispositif de mesure de température (50) est adapté pour mesurer une température de la fenêtre de sortie d'électrons (30).

3. Dispositif de stérilisation par faisceau d'électrons selon la revendication 1 ou 2,
**caractérisé en ce que**
le dispositif de mesure de température (50) comprend au moins deux capteurs de température séparés pour mesurer des températures à des positions distinctes au niveau de la fenêtre de sortie d'électrons (30).

4. Dispositif de stérilisation par faisceau d'électrons selon une des revendications 1 à 3,
**caractérisé en ce que**
la fenêtre de sortie d'électrons (30) comprend une structure de support (32) et une feuille (38) qui est transmissive pour les électrons, et
le dispositif de mesure de température (50) est adapté pour mesurer une température de la structure de support (32) et/ou de la feuille (38).

5. Dispositif de stérilisation par faisceau d'électrons selon une des revendications 1 à 4,
**caractérisé en ce que**
la fenêtre de sortie d'électrons (30) comprend une structure de support (32) destinée à supporter une feuille (38) qui est transmissive pour les électrons,
et
le dispositif de mesure de température (50) est disposé sur et/ou à l'intérieur de la structure de support (32).

6. Dispositif de stérilisation par faisceau d'électrons selon une des revendications 1 à 5,
**caractérisé en ce que**
le dispositif de mesure de température (50) est disposé de manière à mesurer une température dans une partie centrale de la fenêtre de sortie d'électrons (30).

7. Dispositif de stérilisation par faisceau d'électrons selon une des revendications 1 à 6,
**caractérisé en ce que**
le dispositif de mesure de température (50) comprend au moins un thermocouple.

8. Dispositif de stérilisation par faisceau d'électrons selon une des revendications 1 à 7,
**caractérisé en ce que**
le dispositif de mesure de température (50) comprend au moins un capteur de température sans contact (52) destiné à mesurer une température de la fenêtre de sortie d'électrons (30).

9. Procédé de stérilisation d'un récipient d'emballage (60) ou d'une bande de matériau d'emballage par irradiation avec un faisceau d'électrons avec un dispositif de stérilisation par faisceau d'électrons (10) selon une des revendications 1 à 8,
le procédé comprenant les étapes consistant à :
générer un faisceau d'électrons dans un boîtier,
faire sortir (30) le faisceau d'électrons du boîtier par une fenêtre de sortie d'électrons, et
mesurer une température sur une trajectoire de déplacement du faisceau d'électrons en tant que mesure de l'énergie des électrons délivrée par le faisceau d'électrons,
mesurer une température au niveau de chacun d'au moins deux capteurs de température situés à des positions différentes de la fenêtre de sortie d'électrons.
